# EUROPEAN PATENT APPLICATION

(11) **EP 2 174 935 A1**
(43) Date of publication of application: **14.04.2010**
(21) Application number: 09176361.5
(22) Date of filing: 29.06.2005
(51) Int. Cl.: C07D 263/20, A61K 31/421, A61K 31/535

(54) **Linezolid Compositions**

(30) Priority: 29.06.2004 US 584371 P; 30.06.2004 US 584283 P; 12.08.2004 US 601086 P; 17.08.2004 US 602227 P; 07.12.2004 US 633887 P; 24.02.2005 US 656646 P; 24.02.2005 US 656778 P; 05.05.2005 US 678440 P; 24.05.2005 US 684410 P
(62) Divisional of application: 05790033.4
(71) Applicant: Teva Pharmaceutical Industries Ltd, 49131 Petah Tiqva (IL)
(72) Inventor: Aronhime, Judith, 76217 Rehovot (IL); Koltai, Tamas, 42758 Natania (IL); Braude, Viviana, 60920 Kadima (IL); Fine, Serguei, 90100 Qiriat-Arbaa (IL); Niddam, Tamar, 56238 Yehud (IL)
(74) Representative: Russell, Tim

(57) **Abstract**

The present invention provides novel pharmaceutical compositions comprising a crystalline form of linezolid referred to herein as Form IV.

## Description

### Field of the Invention

The present invention relates to a novel pharmaceutical compositions containing linezolid (Form IV).

### Background of the Invention

Linezolid [(S)-N-[[3-(3-Fluoro-4-morpholinyl)phenyl]-2-oxo-5-oxazolidinyl]methyl] acetamide] is an antimicrobial agent. Linezolid is an oxazolidinone, having the empirical formula C₁₆H₂₀FN₃O₄ and the following structure:

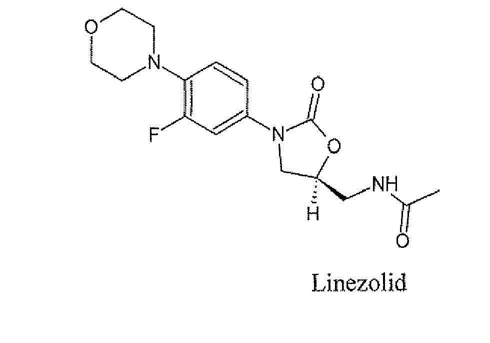

Linezolid is described in The Merck Index (13th edition, Monograph number: 05526, CAS Registry Number: 165800-03-3) as white crystals, with a melting point of 181.5-182.5°. Linezolid, as well as a process for its preparation, is disclosed in U.S. Patent No. 5,688,792 (Example 5), European Patent No. 717738, Israeli Patent No. 110,802, Canadian Patent No. 2,168,560, and International Patent Publication WO 95/07271.

Crystalline Form II linezolid is disclosed in U.S. Patent No. 6,559,305.

Linezolid is marketed in the United States by Pfizer, Inc. as an injection, tablets, and oral suspension under the name ZYVOX®. Its main indications are nosocomial pneumonia, skin and skin-structure infections, and vancomycin-resistant *Enterococcus faecium* infections.

The present invention relates to the solid state physical properties of Linezolid. These properties can be influenced by controlling the conditions under which Linezolid is obtained in solid form. Solid state physical properties include, for example, the flowability of the milled solid. Flowability affects the ease with which the material is handled during processing into a pharmaceutical product. Glidants are often added to improve the flowability of a non-compacted solid composition and to improve the accuracy of dosing. When particles of the powdered compound do not flow past each other easily, a formulation specialist must take that fact into account in developing a tablet or capsule formulation, which may necessitate the use of glidants such as colloidal silicon dioxide, talc, starch or tribasic calcium phosphate.

Crystal forms with platelet shape have better flowability than crystals with needle shape (M. Rouhi, The Right Stuff; Chemical & Engineering News; 24th Feb. 2003). Crystals with plate shape are more advantageous than those with needle shape for injection. A suspension of plate-shaped crystals may be injected through a smaller needle with greater ease than a suspension of needle-shaped crystals. (S.R. Bym et al. Solid-State Chemistry of Drugs 2nd Edition pg.4).

Another important solid state property of a pharmaceutical compound is its rate of dissolution in aqueous fluid. The rate of dissolution of an active ingredient in a patient's stomach fluid can have therapeutic consequences since it imposes an upper limit on the rate at which an orally-administered active ingredient can reach the patient's bloodstream. The rate of dissolution is also a consideration in formulating syrups, elixirs and other liquid medicaments. The solid state form of a compound may also affect its behavior on compaction and its storage stability.

These practical physical characteristics are influenced by the conformation and orientation of molecules in the unit cell, which defines a particular polymorphic form of a substance. These conformational and orientation factors in turn result in particular intramolecular interactions such that different polymorphic forms may give rise to distinct spectroscopic properties that may be detectable by powder X-ray diffraction, solid state ¹³C NMR spectrometry and infrared spectrometry. A particular polymorphic form may also give rise to thermal behavior different from that of the amorphous material or another polymorphic form. Thermal behavior is measured in the laboratory by such techniques as capillary melting point, thermogravimetric analysis (TGA) and differential scanning calorimetry (DSC) and can be used to distinguish some polymorphic forms from others.

The discovery of new polymorphic forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic. There is a need in the art for additional polymorphic forms of Linezolid.

### Summary of the Invention

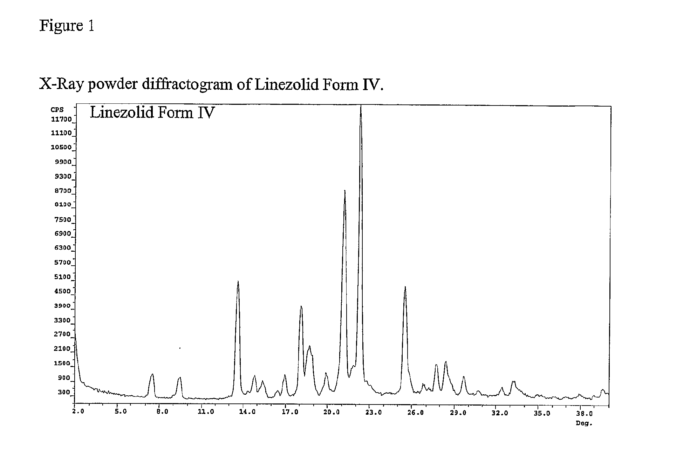

The present invention provides linezolid crystalline Form IV. Form IV is characterized by a powder X-ray diffraction (PXRD) pattern with peaks at about 13.5, 18.0, 21.1, 22.2 and 25.4 ±0.2 degrees 2 theta. Form IV may be further characterized by PXRD peaks at about 7.5, 18.7, 19.9, 27.7 and 28.4 ±0.2 degrees 2 theta. Form IV may be further characterized by a PXRD pattern substantially as depicted in Figure 1.

The crystalline linezolid Form IV of the present invention is polymorphically pure, i.e., contains less than about 10% Form II, preferably less than about 5% Form II, and even more preferably less than about 1% Form II, and most preferably, less than about 0.5% Form II (by weight).

The crystalline linezolid Form IV of the present invention is anhydrous, and is stable against transformation into Form II upon storage at a relative humidity of about 0% to about 100% at room temperature for at least 10 days.

The present invention also provides crystalline linezolid Form IV that is enantiomerically pure with respect to its R-enantiomer, i.e., containing less than about 0.1% area by HPLC of the R-enantiomer, preferably less than about 0.02% area by HPLC of the R-enantiomer.

In one embodiment, the novel crystalline linezolid Form IV is in the form of irregular plate crystals when observed under the microscope.

Another embodiment of the present invention is a novel method of making crystalline linezolid Form IV that comprises spray-drying a solution of linezolid at a temperature of at least 50°C.

Also described in the present invention is another process for preparing crystalline linezolid Form IV comprising the step of heating crystalline linezolid Form II.

Form IV may also be prepared comprising the step of heating a slurry of form II in an aromatic solvent.

Another aspect of the present invention is the preparation of Form II from a slurry of Form IV in water or an organic solvent.

In another embodiment, the crystalline linezolid Form IV provided by the present invention has a preferred maximum particle size of 500 µm.

Another embodiment of the present invention is a pharmaceutical formulation comprising a therapeutically effective amount of crystalline linezolid Form IV, combined with a pharmaceutically acceptable excipient or carrier.

A further embodiment of the present invention is a method for treating a patient suffering from a gram positive bacterial infection, using a formulation containing a therapeutically effective amount of crystalline linezolid Form IV.

### Figures

Figure 1 is a powder X-Ray diffractogram of crystalline linezolid Form IV.

Figure 2a-2c is an FTIR spectrum of crystalline linezolid Form IV obtained with the mineral oil mull technique.

Figure 2d-2f is an FTIR spectrum of crystalline Linezolid Form IV obtaind with the DRIFT technique.

Figure 3 is a DSC thermogram of crystalline linezolid Form IV.

Figure 4a-4d is an FTRaman spectrum of crystalline linezolid Form IV.

Figure 5 shows the needle shape of crystals of crystalline linezolid Form II as seen through a microscope.

Figure 6 shows the plate shape of crystals of crystalline linezolid Form IV as seen through a microscope.

Figure 7 also shows the plate shape of crystals of crystalline linezolid Form IV as seen through a microscope.

Figure 8 is a DSC thermogram of crystalline linezolid Form II.

### Detailed Description of the Invention

The term "spray drying" broadly refers to processes involving breaking up liquid mixtures into small droplets (atomization) and rapidly removing solvent from the mixture. In a typical spray-drying apparatus, there is a strong driving force for evaporation of solvent from the droplets, which may be provided by a heated drying gas. Spray-drying processes and equipment are described in Perry's Chemical Engineer's Handbook, pgs. 20-54 to 20-57 (Sixth Edition 1984).

By way of non-limiting example only, the typical spray-drying apparatus comprises a drying chamber, atomizing means for atomizing a solvent-containing feed into the drying chamber, a source of heated drying gas that flows into the drying chamber to remove solvent from the atomized-solvent-containing feed, an outlet for the products of drying, and product collection means located downstream of the drying chamber. Examples of such apparatuses include Niro Models PSD-1, PSD-2 and PSD-4 (Niro A/S, Soeborg, Denmark). Typically, the product collection means includes a cyclone connected to the drying apparatus. In the cyclone, the particles produced during spray-drying are separated from the drying gas and evaporated solvent, allowing the particles to be collected. A filter may also be used to separate and collect the particles produced by spray-drying. The process of the invention is not limited to the use of such drying apparatuses as described above.

"Therapeutically effective amount" means the amount of a crystalline form that, when administered to a patient for treating a disease or other undesirable medical condition, is sufficient to have a beneficial effect with respect to that disease or condition. The "therapeutically effective amount" will vary depending on the crystalline form, the disease or condition and its severity, and the age, weight, etc., of the patient to be treated. Determining the therapeutically effective amount of a given crystalline form is within the ordinary skill of the art and requires no more than routine experimentation.

The present invention provides crystalline linezolid Form IV, characterized by a powder X-ray diffraction (PXRD) pattern with peaks at about 13.5, 18.0, 21.1, 22.2, and 25.4 ±0.2 degrees 2 theta. Form IV may be further characterized by PXRD peaks at about 7.5, 18.7, 19.9, 27.7, and 28.4 ±0.2 degrees 2 theta. Form IV may be further characterized by a PXRD pattern substantially as depicted in Figure 1.

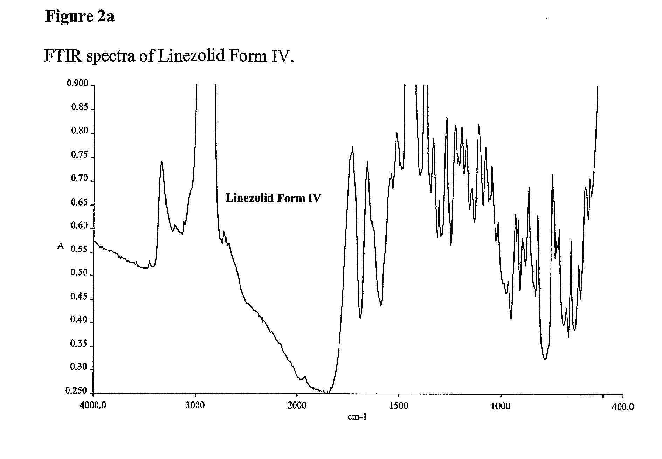

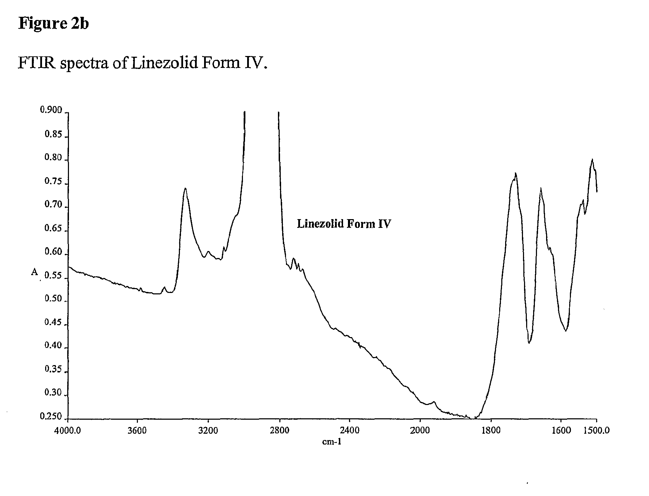

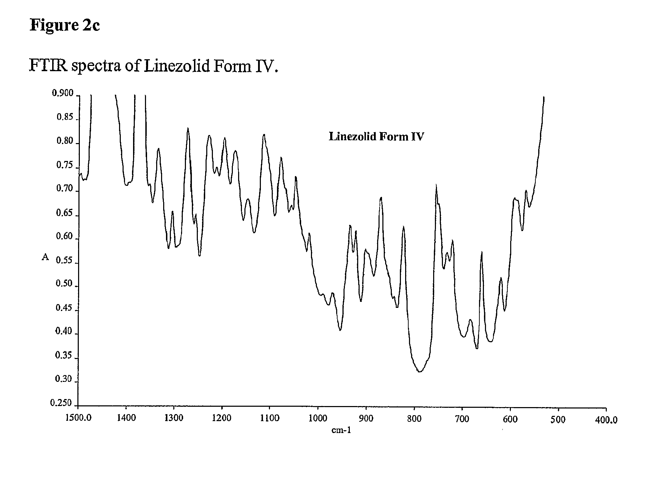

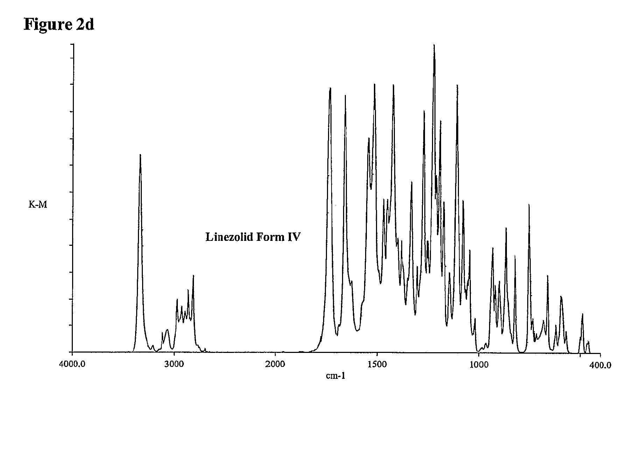

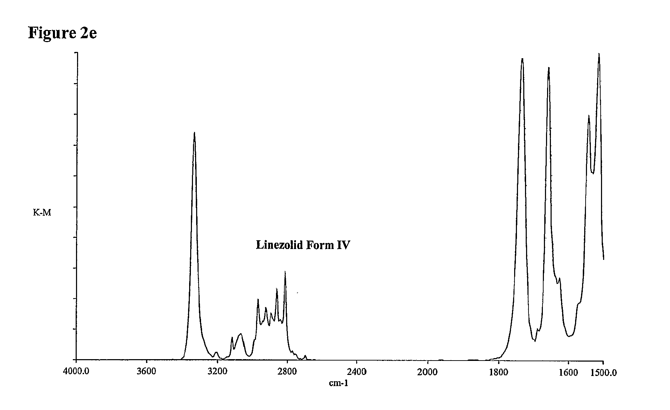

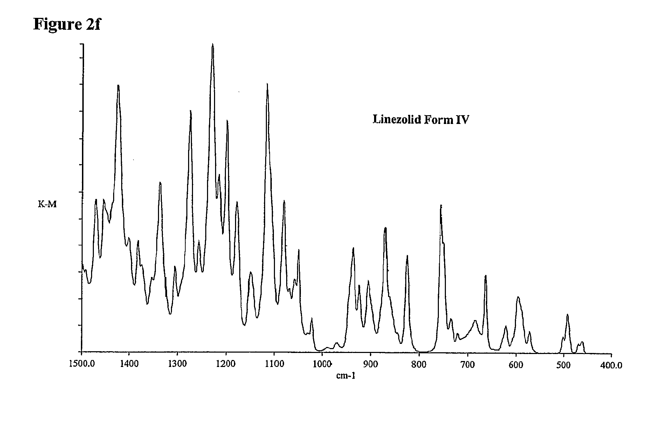

Crystalline linezolid Form IV may be further characterized by an FTIR spectrum with peaks at about 3333, 2817, 1732, 1661, 1540, 1515, 1335, 1256, 1229, 1200, 1213, 1148, 1080, 1059, 1050, 903, 825, 755, and 662 cm⁻¹. Crystalline linezolid Form IV may be further characterized by an FTIR spectrum substantially as depicted in Figure 2a-2c or in Figure 2d-2f. The most characteristic FTIR peaks are 2817, 1335, 1229, 1200, and 662 cm⁻¹.

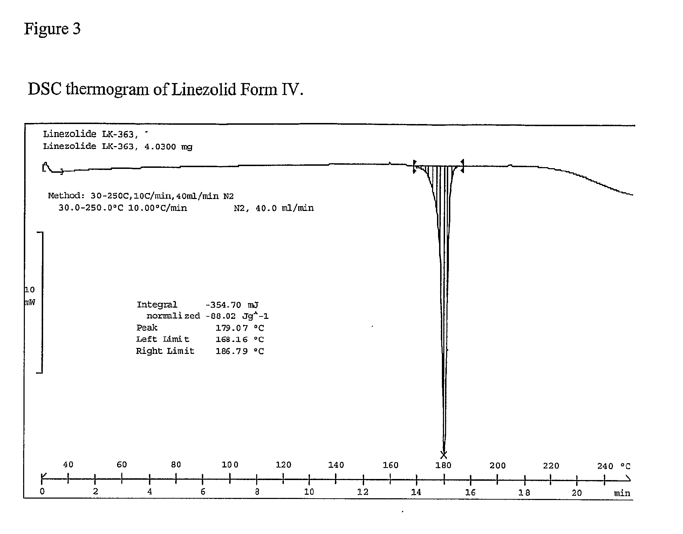

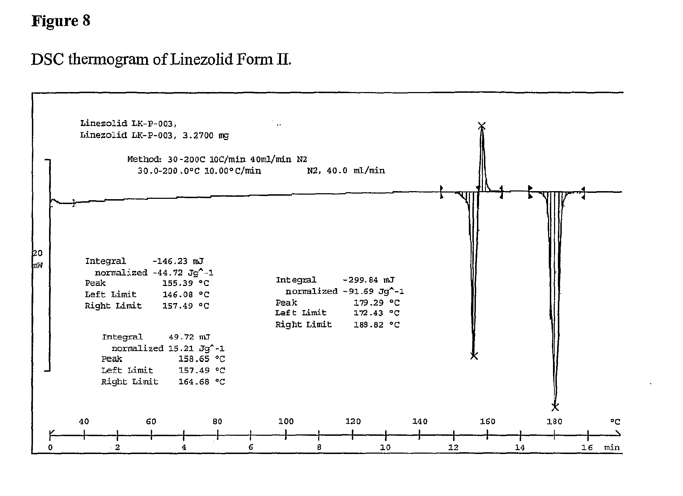

Crystalline linezolid Form IV may also be characterized by a differential scanning calorimetry (DSC) thermogram substantially as depicted in Figure 3. Form IV has a characteristic DSC thermogram compared to the DSC thermogram of Form II (Figure 8). Form II has a sharp endothermic peak around 155°C, followed by a sharp exothermic peak at around 160°C, and another sharp endothermic peak at around 180°C, which corresponds to the final melting of linezolid. Form IV has only one sharp endothermic peak, which corresponds to the final melting of linezolid.

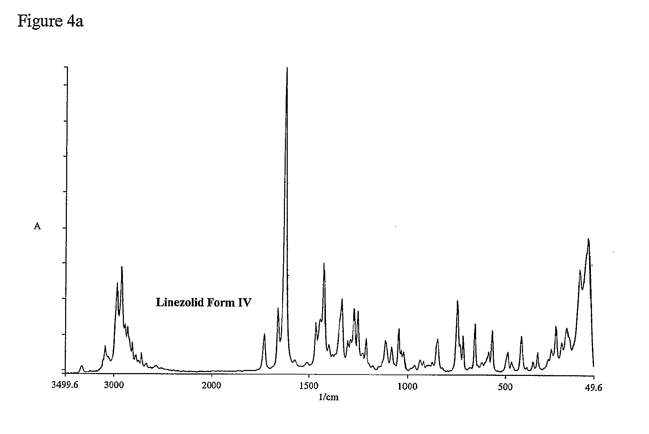

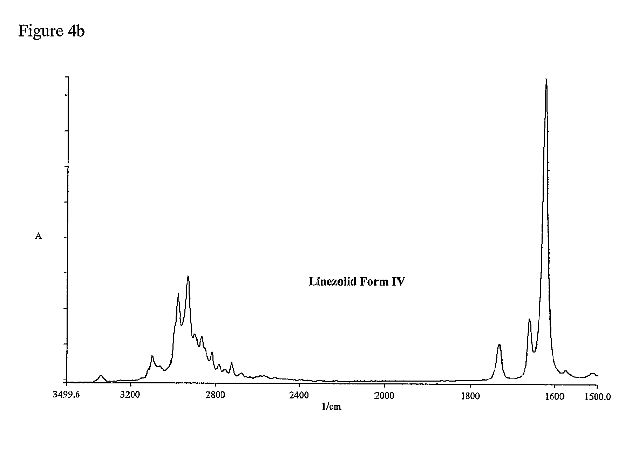

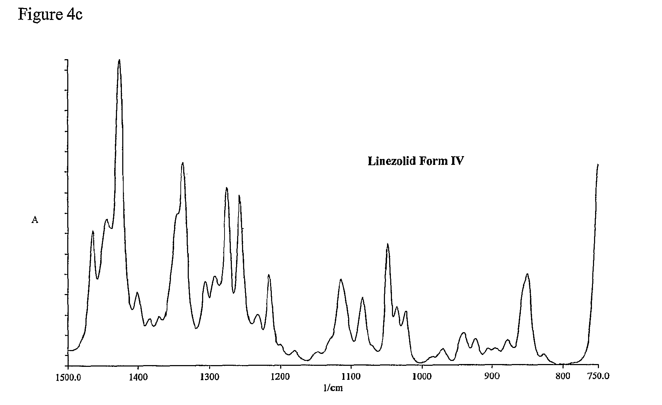

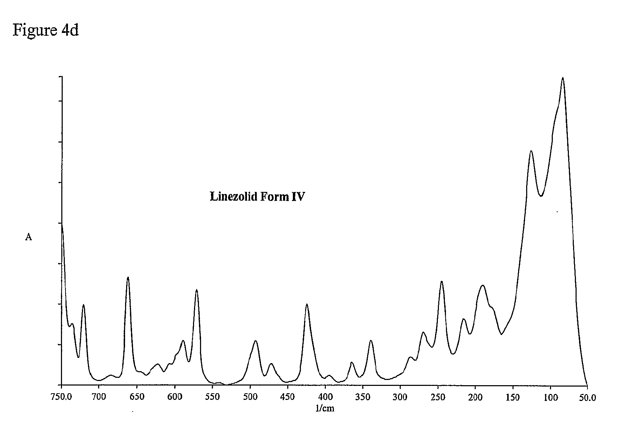

Crystalline linezolid Form IV may be further characterized by an FTRaman spectrum having peaks at about 2975, 2930, 2865, 1083, and 750 cm⁻¹. Crystalline linezolid Form IV may be further characterized by an FTRaman spectrum substantially as depicted in Figure 4a-4d.

The invention also provides crystalline linezolid Form IV, which has about 0.1% water by weight. Form IV may also be characterized by a weight loss measured by thermal gravimetric analysis (TGA) of about 0.1% by weight. This embodiment of Form IV is anhydrous. Thus, the present invention includes crystalline linezolid Form IV which has about 0.1 % or less water by weight.

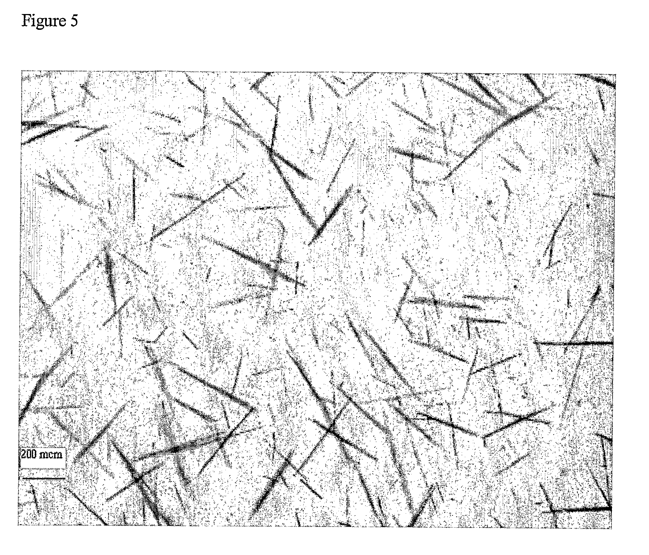

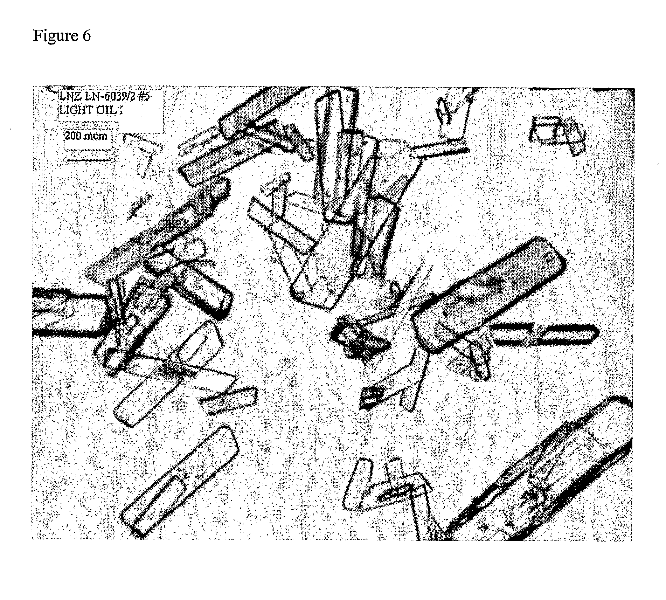

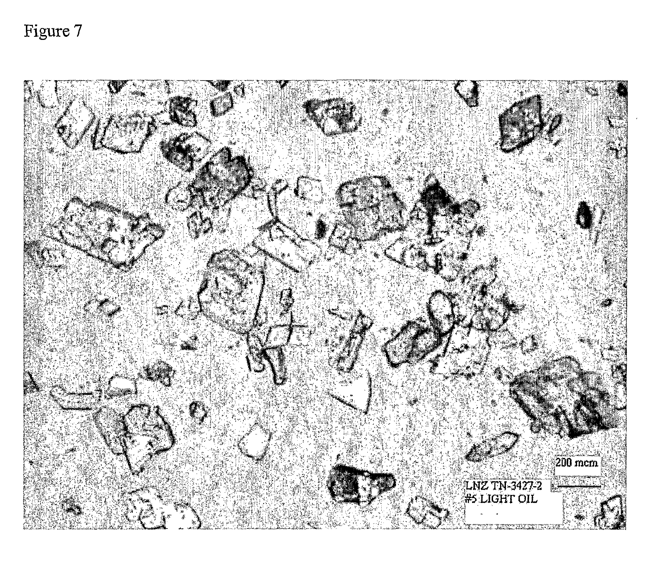

The crystalline linezolid Form IV of the present invention can be made in the form of irregular plate crystals. The previously known crystalline linezolid Form II forms needle shaped crystals. The flowability of crystals with an irregular plate shape of the present invention is much better than the flowability of crystals with needle shape. Another advantage of the irregular plate crystals is that they are easier to work with than needle shaped linezolid of the prior art. The bulk properties of the crystalline linezolid Form IV of the present invention are advantageous compared to those of the prior art linezolid. The difference between the needle-shaped crystals of Form II and the plate-shaped crystals of Form IV can be appreciated by a comparison of Figure 5 (Form II) with Figure 6 (Form IV) or Figure 7 (also Form IV).

The crystalline linezolid Form IV provided in the present invention is enantiomerically pure. Enantiomerically pure linezolid Form IV contains the unwanted R-enantiomer at a level of less than about 0.1% area by HPLC. Preferably, the enantiomerically pure linezolid Form IV of the present invention contains less than about 0.02% area by HPLC of the R-enantiomer.

The crystalline linezolid Form IV of the present invention is also polymorphically pure. Thus, it contains less than about 10% Form II, preferably less than about 5% Form II, and even more preferably less than about 1% Form II, and most preferably, less than about 0.5% Form II (by weight).

The present invention provides a novel method of making crystalline linezolid Form IV by spray-drying a solution of linezolid at a temperature of at least 50°C. This method comprises dissolving linezolid in a polar organic solvent at about room temperature, pumping the obtained solution into a spray dryer and contacting it with a nitrogen gas at a temperature of at least 50°C until Form IV is obtained. Preferably, the polar organic solvent is methanol.

The present invention provides another process for preparing crystalline linezolid Form IV comprising the step of heating crystalline linezolid Form II to a temperature of at least 85°C, preferably for at least one hour.

Form IV may also be prepared by heating a slurry of Form II in an organic solvent. The slurry is produced by combining Form II with an aromatic solvent at room temperature, followed by heating for one hour to induce partial dissolution. The partially clear slurry is then filtered and the obtained solid is dried, providing crystals that were analyzed by PXRD and shown to be linezolid Form IV.

Preferred organic solvents are aromatic solvents and more preferably, xylene and toluene. Another preferred solvent is a silicon fluid.

Enantiomerically pure Form IV is prepared by slurrying Form II in an organic solvent, as described above, and using Form II that contains a maximum of 0.1% of the R-enantiomer. Preferably, the Form II contains less than about 0.02% of the R-enantiomer.

Form II and the solvents used in the slurrying processes are preferably combined at 25°C, providing a slurry that is heated, preferably to reflux, when an aromatic solvent is used, and more preferably, to 140°C, when a silicon fluid is used. The isolated linezolid Form IV is dried, preferably in an oven, preferably by heating to a temperature of 50°C.

The invention encompasses a process for the transformation of Form IV into Form II comprising combining Form IV at room temperature with water or with an organic solvent selected from the group consisting of a ketone, a straight or branched C₁-C₄ alcohol, an ester, an aromatic solvent, and a nitrile, to obtain a slurry, and maintaining the slurry for at least about one hour. The slurry is then filtered, providing crystals of Form II, which are then dried and analyzed by PXRD.

The temperature of the reaction is preferably room temperature. The slurry is maintained for at least about one hour, preferably for about one hour to about 24 hours.

The stability of linezolid Form IV was tested by storing it for 10 days at a relative humidity of between 0 and 100% at room temperature. The results are summarized in Table 1.

**TABLE 1**

| Relative humidity [%] | Solvent content [%] | Crystal Form |
|---|---|---|
| As is | 0.1 | IV |
| 0 | 0.1 | IV |
| 20 | 0.1 | IV |
| 40 | 0.1 | IV |
| 60 | 0.1 | IV |
| 80 | 0.1 | IV |
| 100 | 0.1 | IV |

Form IV has proven to be stable and non-hygroscopic between 0-100 % RH.

The long term stability of linezolid Form IV was tested by storing it at room temperature and under accelerated conditions at 40°C and 55°C. The results are summarized in Table 2.

**Table 2**

| Results | | | |
|---|---|---|---|
| Time | 25°C | 40°C | 55°C |
| As is | Form IV | NA | NA |
| 1M | Form IV | Form TV | Form IV |
| 2M | Form IV | Form IV | Form IV |
| 3M | Form IV | Form TV | Form IV |

Form IV has proven to be stable at 25°C, 40°C, and 55°C for a minimum of 3 months.

The particle size distribution (PSD) of the active ingredient is one of the key parameters of a formulation. For measuring particle size, the following main methods may be employed: sieves, sedimentation, electrozone sensing (coulter counter), microscopy, Low Angle Laser Light Scattering (LALLS). The crystalline linezolid Form IV of the present invention has a preferred maximum particle size of 500 µm. Preferably, the particle size is less than 300 µm, less than 200 µm, less than 100 µm, or even less than 50 µm.

Another embodiment of the present invention is a pharmaceutical formulation comprising a therapeutically effective amount of crystalline linezolid Form IV, combined with a pharmaceutically acceptable excipient or carrier.

Another embodiment of the present invention is a method for treating a patient suffering from a gram positive bacterial infection, comprising the step of administering to the patient the pharmaceutical formulation comprising a therapeutically effective amount of crystalline linezolid Form IV.

The crystalline linezolid Form IV of the present invention used to prepare pharmaceutical formulations may be substantially pure with respect to other crystalline forms, i.e., the pharmaceutical formulations may contain less than about 10%, preferably less than about 5%, and even more preferably less than about 1% (by weight) of other crystalline forms of linezolid. In particular, the pharmaceutical formulations may contain less than about 10%, preferably less than about 5%, and more preferably less than about 1%, and even more preferably less than 0.5% (by weight) of crystalline linezolid Form II. In certain embodiments, the pharmaceutical formulations may contain less than about 10%, preferably less than about 5%, and even more preferably less than about 1% (by weight) of amorphous linezolid.

In other embodiments, pharmaceutical formulations of the present invention may contain crystalline linezolid Form IV in a mixture with other forms of linezolid.

In addition to the active ingredient(s), the pharmaceutical formulations of the present invention may contain one or more excipients. Excipients are added to the formulation for a variety of purposes.

Diluents may be added to the formulations of the present invention. Diluents increase the bulk of a solid pharmaceutical composition, and may make a pharmaceutical dosage form containing the composition easier for the patient and care giver to handle. Diluents for solid compositions include, for example, microcrystalline cellulose (e.g., AVICEL®), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g., EUDRAGIT®), potassium chloride, powdered cellulose, sodium chloride, sorbitol, and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g., carbopol), carboxymethylcellulose sodium, dextrin, ethyl cellulose, gelatin, guar gum, hydrogenated vegetable oil, hydroxyethyl cellulose, hydroxypropyl cellulose (e.g., KLUCEL®), hydroxypropyl methyl cellulose (e.g., METHOCEL®), liquid glucose, magnesium aluminum silicate, maltodextrin, methylcellulose, polymethacrylates, povidone (e.g., KOLLIDON®, PLASDONE®), pregelatinized starch, sodium alginate, and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g., AC-DI-SO®, PRIMELLOSE®), colloidal silicon dioxide, croscarmellose sodium, crospovidone (e.g., KOLLIDON®, POLYPLASDONE®), guar gum, magnesium aluminum silicate, methyl cellulose, microcrystalline cellulose, polacrilin potassium, powdered cellulose, pregelatinized starch, sodium alginate, sodium starch glycolate (e.g., EXPLOTAB®), and starch.

Glidants can be added to improve the flowability of a non-compacted solid composition and to improve the accuracy of dosing. Excipients that may function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol, and tartaric acid.

Solid and liquid compositions may also be dyed using any pharmaceutically acceptable colorant to improve their appearance and/or facilitate patient identification of the product and unit dosage level.

The present invention is not intended to encompass true solutions of crystalline linezolid Form IV whereupon the crystal structure of the novel Form IV and the properties that characterize the novel crystalline form of the present invention are lost. However, the use of the novel form to prepare such solutions (e.g., so as to deliver linezolid in a liquid pharmaceutical formulation) is considered to be within the contemplation of the invention.

In liquid pharmaceutical compositions prepared using Form IV of the present invention, Form IV and any other solid excipients are dissolved or suspended in a liquid carrier such as water, vegetable oil, alcohol, polyethylene glycol, propylene glycol, or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol, and cetyl alcohol.

Liquid pharmaceutical compositions may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth, and xanthan gum.

Sweetening agents such as sorbitol, saccharin, sodium saccharin, sucrose, aspartame, fructose, mannitol, and invert sugar may be added to improve the taste.

Preservatives and chelating agents such as alcohol, sodium benzoate, butylated hydroxyl toluene, butylated hydroxyanisole, and ethylenediamine tetraacetic acid may be added at levels safe for ingestion to improve storage stability.

A liquid composition may also contain a buffer such as guconic acid, lactic acid, citric acid or acetic acid, sodium guconate, sodium lactate, sodium citrate, or sodium acetate. Selection of excipients and the amounts used may be readily determined by the formulation scientist based upon experience and consideration of standard procedures and reference works in the field.

The solid compositions of the present invention include powders, granulates, aggregates and compacted compositions. The dosages include dosages suitable for oral, buccal, rectal, parenteral (including subcutaneous, intramuscular, and intravenous), inhalant, and ophthalmic administration. Although the most suitable administration in any given case will depend on the nature and severity of the condition being treated, the most preferred route of the present invention is oral. The dosages may be conveniently presented in unit dosage form and prepared by any of the methods well-known in the pharmaceutical arts.

Dosage forms include solid dosage forms like tablets, powders, capsules, suppositories, sachets, troches and lozenges, as well as liquid syrups, suspensions and elixirs.

The dosage of GEODON may be used as guidance. The oral dosage form of the present invention is preferably in the form of an oral capsule having a dosage of about 10 mg to about 160 mg, more preferably from about 20 mg to about 80 mg, and most preferably capsules of 20,40, 60 and 80 mg. Daily dosages may include 1, 2, or more capsules per day.

The dosage form of the present invention may be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

A composition for tableting or capsule filling may be prepared by wet granulation. In wet granulation, some or all of the active ingredients and excipients in powder form are blended and then further mixed in the presence of a liquid, typically water, that causes the powders to clump into granules. The granulate is screened and/or milled, dried and then screened and/or milled to the desired particle size. The granulate may then be tableted, or other excipients may be added prior to tableting, such as a glidant and/or a lubricant.

A tableting composition may be prepared conventionally by dry blending. For example, the blended composition of the actives and excipients may be compacted into a slug or a sheet and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention may comprise any of the aforementioned blends and granulates that were described with reference to tableting, however, they are not subjected to a final tableting step.

The active ingredient and excipients may be formulated into compositions and dosage forms according to methods known in the art.

The crystalline linezolid Form IV of the present invention may be used in pharmaceutical formulations or compositions as a single active ingredient or as a mixture together with other crystalline forms of linezolid or with amorphous linezolid. However, it is preferred that the pharmaceutical formulations or compositions of the present invention contain 25-100% by weight, especially 50-100% by weight, of crystalline linezolid Form IV, based on the total amount of linezolid in the formulation or composition. Preferably, such an amount of crystalline linezolid Form IV is 75-100% by weight, especially 90-100% by weight. Highly preferred is an amount of 95-100% by weight.

Having described the invention with reference to certain preferred embodiments, other embodiments will become apparent to one skilled in the art from consideration of the specification. The invention is further defined by reference to the following examples describing in detail the preparation of the composition and methods of use of the invention. It will be apparent to those skilled in the art that many modifications, both to materials and methods, may be practiced without departing from the scope of the invention.

### Examples

Microscopic samples were prepared in mineral oil suspension and placed between slides.

Powder X-ray diffraction Powder X-ray diffraction data were obtained by methods known in the art using a SCINTAG® powder X-ray diffractometer model X'TRA® equipped with a solid state detector. Copper radiation of 1.5418 Å was used. A round aluminum sample holder with round zero background quartz plate was used, with cavity of 25(diameter)* 0.5(depth) mm. The obtained characteristic peaks were in the range of 2-40 degrees two theta.

DSC analysis was done using a Mettler 821 Star^{c}. The weight of the samples was about 5 mg; the samples were scanned at a rate of 10°C/min from 30°C to 250°C. The oven was constantly purged with nitrogen gas at a flow rate of 40 ml/min. Standard 70 µl alumina crucibles covered by lids with 1 hole were used.

IR analysis was done using a Perkin Elmer SPECTRUM ONE FT-IR spectrometer in DRIFT mode or using mineral oil mull technique. The samples in the 4000-400 cm⁻¹ interval were scanned 16 times with 4.0 cm⁻¹ resolution.

The FTRaman analysis was performed by Bruker RFS-100/S Raman spectrometer. The scanning parameters were:
Range: 3500 to 50 cm⁻¹;
Aperture Setting: 10.0mm;
Low Pass Filter 16: 1kHz;
Source Setting Laser: 9394.0 cm⁻¹, 1600 mW;
Raman Laser Power: 500 mW;
Scanner: Velocity 5.0 at 4 kHz;
Sample Scans: 100; and
Resolution: 4.0 cm⁻¹,

### Example 1 - preparation of crystalline linezolid Form IV

A flask containing 5.5 g of (S)-N-(4-morpholinyl-3-fluorophenyl)-2-oxo-5-oxazolidinyl-methyl amine and 20 ml ammonium hydroxide in 200 ml toluene was mixed at 25°C. Triethyl amine (2 equivalents) was added. The mixture was cooled to 3°C and acetic anhydride (2.5 equivalents) was added dropwise. The reaction mixture was brought to RT. Linezolid that precipitated from the reaction mixture was filtered. The wet crystals were analyzed by PXRD and shown to be crystalline linezolid Form IV.

### Example 2 - Procedure for the preparation of crystalline linezolid Form IV

This example was carried out on a Buchi Mini Spray Dryer B-290. 5 g of linezolid was dissolved in methanol (300 ml) at room temperature. The obtained solution was pumped into the spray dryer. The nitrogen was at an inlet temperature of 50°C. The evaporated solvent and nitrogen exited the spray dryer at 35°C. The obtained sample was analyzed by PXRD and shown to be linezolid Form IV.

### Example 3 - preparation of crystalline linezolid Form IV by heating crystalline linezolid Form II

Crystalline linezolid Form IV may be prepared by heating crystalline linezolid Form II to a temperature higher than 85°C. Crystalline linezolid Form IV is obtained by keeping crystalline linezolid Form II at a temperature of between 85-175°C. As an example, crystalline linezolid Form II was kept at a temperature of 170°C during one hour. The polymorphic form of crystalline linezolid Form II was changed to crystalline linezolid Form IV. The crystalline linezolid Form IV obtained at 170°C was chemically stable.

### Example 4 - preparation of crystalline linezolid Form IV by reslurrying Form II in xylene

Crystalline (S)-N-(4-morpholinyl-3-fluorophenyl)-2-oxo-5-oxazolidinyl-methyl acetamide (Form II having an (R)-Linezolid content of 0.02%) (2.2 g) was mixed with xylene (20 ml) at 25°C. The mixture was heated to reflux and stirred for one hour to induce partial dissolution. The clearer slurry was then filtered and the obtained crystals were dried at 50°C in an oven overnight. The crystals obtained were analyzed by PXRD and shown to be Linezolid Form IV, with the same enantiomeric content.

### Example 5 - preparation of crystalline linezolid Form IV by reslurrying Form II in toluene

Crystalline (S)-N-(4-morpholinyl-3-fluorophenyl)-2-oxo-5-oxazolidinyl-methyl acetamide (Form II having an (R)-Linezolid content of 0.02%) (2.2 g) was mixed with toluene (20 ml) at 25°C. The mixture was heated to reflux and stirred for one hour to induce partial dissolution. The clearer slurry was then filtered and the obtained crystals were dried at 50°C in an oven overnight. The crystals obtained were analyzed by PXRD and shown to be Linezolid Form IV, with the same enantiomeric content.

### Example 6 - preparation of crystalline linezolid Form IV by reslurrying Form II in a silicon fluid

Crystalline (S)-N-(4-morpholinyl-3-fluorophenyl)-2-oxo-5-oxazolidinyl-methyl acetamide (Form II having an (R)-Linezolid content of 0.02%) (2.2 g) was mixed with a silicon fluid (20 ml) at 25°C. The mixture was heated to 140°C and stirred for one hour to induce partial dissolution. The clearer slurry was then filtered and the obtained crystals were dried at 50°C in an oven overnight. The crystals obtained were analyzed by PXRD and shown to be Linezolid Form IV, with the same enantiomeric content

### Example 7 - preparation of crystalline linezolid Form II from a slurry of Form IV

A slurry of linezolid Form IV was produced by combining linezolid Form IV (2.0 g) with one of the solvents listed below, at room temperature. The slurry was then stirred for 1 to 24 hours, followed by filtering and drying the obtained crystals. The crystals were analyzed by PXRD and shown to be Form II of Linezolid, as summarized in the following Table 3:

**Table 3**

| **Solvent/s** | **V** | **T** | **t** | **XRD** | |
|---|---|---|---|---|---|
| | **mg/ml** | **(°C)** | **(h)** | | **Form** |
| acetone | 5 | RT | 2 | d | II |
| EtOH 95% | 5 | RT | 2 | w | II |
| | | | | d | II |
| EtOH 95% | 10 | RT | 1 | d | II |
| EtOAc | 5 | RT | 2 | w | II |
| | | | | d | II |
| toluene | 5 | RT | 2 | w | IV |
| | | | | d | IV |
| ACN | 5 | RT | 2 | w | II |
| | | | | d | II |
| acetone | 5 | RT | 2 | w | II |
| | | | | d | II |
| acetone | 5 | RT | 24 | w | II |
| | | | | d | II |
| IPA | 5 | RT | 24 | w | II |
| | | | | d | II |
| H₂O | 10 | RT | 1 | d | II+∼10%IV |
| | | | | | |

Further aspects and features of the present invention are set out in the following numbered clauses.
1. Crystalline linezolid characterized by data selected from the group consisting of:
   a) a powder X-ray diffraction pattern with peaks at about 13.5, 18.0, 21.1, 22.2, and 25.4 ±0.2 degrees 2 theta;
   b) an FTIR spectrum with peaks at about 2817, 1335, 1229, 1200, and 662 cm⁻¹; and
   c) an FTRaman spectrum with peaks at about 2975, 2930, 2865, 1083, and 750 cm⁻¹.
2. The crystalline linezolid of clause 1 characterized by an FTIR spectrum with peaks at about 3333, 2817, 1732, 1661, 1540, 1515, 1335, 1256, 1229, 1200, 1213, 1148, 1080, 1059, 1050, 903, 825, 755, and 662 cm⁻¹.
3. The crystalline linezolid of clause 1 characterized by a powder X-ray diffraction pattern with peaks at about 7.5, 13.5, 18.0, 18.7, 19.9, 21.1, 22.2, 25.4, 27.7, and 28.4 ±0.2 degrees 2 theta.
4. The crystalline linezolid of clause 1 which has a powder X-ray diffraction pattern substantially as depicted in Figure 1 or an FTIR spectrum substantially as depicted in Figure 2a-2c or in Figure 2d-2f.
5. The crystalline linezolid of clause 1 which has a DSC thermogram substantially as depicted in Figure 3 or an FTRaman spectrum substantially as depicted in Figure 4a-4d.
6. The crystalline linezolid of clause 1, having a plate-shaped morphology.
7. The crystalline linezolid of clause 1 which has about 0.5 % or less water by weight.
8. The crystalline linezolid of clause 1 having a content of less than 0.5% (w/w) of the R-enantiomer of linezolid.
9. The crystalline linezolid of clause 1, containing less than about 10% of crystalline Form II.
10. The crystalline linezolid of clause 9, containing less than about 5% of crystalline Form II.
11. The crystalline linezolid of clause 10, containing less than about 0.5% of crystalline Form II.
12. The crystalline linezolid of clause 1 which is stable against transformation into Form II upon storage at a relative humidity of about 0% to about 100% at room temperature for at least 10 days.
13. A method of preparing the crystalline linezolid of clause 1, comprising:
   a) dissolving linezolid in a polar organic solvent to form a linezolid solution;
   b) spray-drying the linezolid solution at a temperature of at least 50°C
14. The method of clause 13 where the dissolving is carried out at room temperature
15. The method of clause 13 where the polar organic solvent is methanol.
16. The method of clause 13 where the spray-drying is carried out with nitrogen gas.
17. A process for preparing the crystalline linezolid of clause 1, comprising heating crystalline Linezolid Form II to a temperature of at least 85°C for at least one hour.
18. A process for preparing crystalline linezolid Form II comprising:
   a) combining crystalline linezolid Form IV with a solvent at room temperature to form a slurry;
   b) stirring the slurry for 1 to 24 hours to obtain crystalline material;
   c) filtering and drying the crystalline material to obtain crystalline linezolid Form II.
19. The process of clause 18 where the solvent is selected from the group consisting of: acetone, EtOH 95%, EtOAc, toluene, CAN, IPA, and H₂O.
20. A process for preparing crystalline linezolid Form IV comprising:
   a) combining crystalline linezolid Form II with a solvent at room temperature to form a slurry;
   b) heating the slurry;
   c) filtering the heated slurry to obtain crystalline linezolid Form IV.
21. The process of clause 20 where the solvent is selected from the group consisting of: xylene, toluene, and a silicon fluid.
22. A pharmaceutical composition comprising a therapeutically effective amount of the crystalline linezolid of clause 1 and a pharmaceutically acceptable excipient or carrier.
23. A method of treating a patient with a gram-positive bacterial infection comprising administering to the patient the pharmaceutical composition of clause 22.

## Claims

1. A pharmaceutical composition prepared by a wet granulation process in the form of a tablet or a capsule comprising crystalline linezolid **characterized by** data selected from the group consisting of:
i) a powder X-ray diffraction pattern with peaks at about 13.5, 18.0, 21.1, 22.2, and 25.4 ± 0.2 degrees 2 theta;
ii) an FTIR spectrum with peaks at about 2817, 1335, 1229, 1200, and 662 cm^{- 1}; and
iii) an FTRaman spectrum with peaks at about 2975, 2930, 2865, 1083, and 750 cm⁻¹,
and a pharmaceutically acceptable excipient.

2. The pharmaceutical composition of claim 1, wherein the process comprises blending some or all of the linezolid and excipient in powder form, then mixing the blend in the presence of a liquid that causes the powders to clump into granules, and then the granulate is tableted.

3. The pharmaceutical composition of claim 2, wherein the liquid is water.

4. The pharmaceutical composition of claim 2 or claim 3, wherein the prior to tableting further excipients are added.

5. The pharmaceutical composition of any one of the preceding claims, wherein the pharmaceutically acceptable excipient is povidone.

6. A pharmaceutical composition prepared by a dry granulation process in tablet form comprising crystalline linezolid **characterized by** data selected from the group consisting of:
i) a powder X-ray diffraction pattern with peaks at about 13.5, 18.0, 21.1, 22.2, and 25.4 ± 0.2 degrees 2 theta;
ii) an FTIR spectrum with peaks at about 2817, 1335, 1229, 1200, and 662 cm^{- 1}; and
iii) an FTRaman spectrum with peaks at about 2975, 2930, 2865, 1083, and 750 cm⁻¹,
and a pharmaceutically acceptable excipient.

7. The pharmaceutical composition of claim 6, wherein the dry granulation process comprises blending the linezolid and excipient, compacting the blend into slugs or a sheet, and comminuting into compacted granules, and then compressing the compacted granules into a tablet.

8. The pharmaceutical composition of claim 6 or claim 7, wherein the pharmaceutically acceptable excipient is povidone.

9. A pharmaceutical composition prepared by a direct compression process in the form of a tablet comprising crystalline linezolid **characterized by** data selected from the group consisting of:
i) a powder X-ray diffraction pattern with peaks at about 13.5, 18.0, 21.1, 22.2, and 25.4 ± 0.2 degrees 2 theta;
ii) an FTIR spectrum with peaks at about 2817, 1335, 1229, 1200, and 662 cm^{- 1}_{;} and
iii) an FTRaman spectrum with peaks at about 2975, 2930, 2865, 1083, and 750 cm⁻¹,
and a pharmaceutically acceptable excipient.

10. The pharmaceutical composition of claim 9, wherein the pharmaceutically acceptable excipient is povidone.
